(19) Europäisches Patentamt
European Patent Office
Office européen des brevets

(11) **EP 0 914 516 B1**

(12) **EUROPÄISCHE PATENTSCHRIFT**

(45) Veröffentlichungstag und Bekanntmachung des Hinweises auf die Patenterteilung:
**01.10.2003 Patentblatt 2003/40**

(21) Anmeldenummer: **97933676.5**

(22) Anmeldetag: **14.07.1997**

(51) Int Cl.7: **D06P 5/04**, D06P 5/06, D06P 1/62

(86) Internationale Anmeldenummer:
**PCT/EP97/03753**

(87) Internationale Veröffentlichungsnummer:
**WO 98/003725 (29.01.1998 Gazette 1998/04)**

(54) **VERFAHREN ZUR REDUKTIVEN NACHREINIGUNG VON POLYESTERHALTIGEN TEXTILIEN**

PROCESS FOR THE REDUCTIVE POST-CLEANING OF POLYESTER-CONTAINING TEXTILES

PROCEDE DE NETTOYAGE ULTERIEUR REDUCTEUR POUR TEXTILES CONTENANT DES POLYESTERS

(84) Benannte Vertragsstaaten:
**DE ES FR GB IT**

(30) Priorität: **23.07.1996 DE 19629453**

(43) Veröffentlichungstag der Anmeldung:
**12.05.1999 Patentblatt 1999/19**

(73) Patentinhaber: **BASF AKTIENGESELLSCHAFT
67056 Ludwigshafen (DE)**

(72) Erfinder:
- **BECKMANN, Eberhard
D-67435 Neustadt (DE)**
- **KRÜGER, Rudolf
D-67273 Weisenheim (DE)**
- **KARL, Ulrich
D-67061 Ludwigshafen (DE)**
- **TRITSCHLER, Claus
D-68542 Heddesheim (DE)**

(56) Entgegenhaltungen:
**DE-B- 2 744 607** **FR-A- 2 379 645**
**GB-A- 1 139 558** **US-A- 4 400 174**

- **PATENT ABSTRACTS OF JAPAN vol. 018, no. 058 (C-1159), 31.Januar 1994 & JP 05 272075 A (NAGASE SANGYO KK), 19.Oktober 1993, in der Anmeldung erwähnt**
- **PATENT ABSTRACTS OF JAPAN vol. 014, no. 290 (C-0731), 22.Juni 1990 & JP 02 091285 A (SANYO CHEM IND LTD), 30.März 1990, in der Anmeldung erwähnt**
- **T.M. BALDWINSON: "Some Observations on Colour Fastness to Washing with Particular Reference to Disperse Dyes on Polyester" JOURNAL OF THE SOCIETY OF DYERS AND COLOURISTS., Bd. 91, Nr. 4, April 1975, BRADFORD GB, Seiten 97-102, XP002048091**
- **DR. S. HEIMANN: "Neue Möglichkeit zur Nachbehandlung gefärbter Polyesterfasern" CHEMIEFASERN TEXTIL-INDUSTRIE., Bd. 30, Nr. 11, November 1980, FRANKFURT AM MAIN DE, Seiten 898-901, XP002048092**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 914 516 B1

Printed by Jouve, 75001 PARIS (FR)

## Beschreibung

Die Erfindung beschreibt ein Verfahren zur reduktiven Nachreinigung von gefärbten polyesterhaltigen Textilien.

In der Küpenfärberei werden üblicherweise die cellulosehaltigen Textilien eingefärbt, indem geeignete Farbstoffe im stark alkali-schem Milieu durch Reduktionsmittel in ihre lösliche Leukoform überführt werden, welche nach Eindringen in die Faser durch Luftoxidation wieder in den unlöslichen Farbstoff überführt wird. Als Reduktionsmittel kommt dabei meist Na-Dithionit zur Anwendung, doch ist auch der Einsatz von Sulfinaten aus der GB 829 177 für verwandte Druckanwendungen bekannt. Mischungen aus Dithionit mit Sulfinaten bzw. Sulfonaten werden nach den Schriften US 3 265 459 bzw. US 3 645 665 und US 3 798 172 als Reduktionsmittel zur Zellulosefärbung eingesetzt.

Die Färbung polyesterhaltiger Textilien erfolgt dagegen ausschließlich im sauren Milieu. Dabei wird entweder nach dem Hochtemperatur-Auszieh-(HT-AZ)Verfahren, dem Thermosolverfahren oder dem Heißdampffixierungsverfahren vorgegangen. Bei ersterem Prozeß wird die Textilie im Färbebad bei einem pH-Wert von etwa 3 - 6 und einer Temperatur von ca. 120 - 140°C im Druckkessel gefärbt. Durch diese Behandlung diffundiert der Dispersionsfarbstoff in die erweichte Polyesterfaser ein und liegt dann molekular verteilt in der Polymermatrix vor. Nach Beendigung des Färbeschrittes erfolgt gewöhnlich eine Vorreinigung mit Frischwasser und schließlich eine alkalische, reduktive Nachreinigung im Waschbad, wobei hier üblicherweise Na-Dithionit und Ätznatron in wäßriger Lösung eingesetzt werden.

Sowohl im Thermosolverfahren als auch im Heißdampffixierungsprozeß wird ebenfalls nach der Vorreinigung eine alkalische, reduktive Nachreinigung vorgenommen.

Wie im HT-AZ-Verfahren wird auch im Thermosol- sowie Heißdampffixierungsverfahren als NBM zum überwiegenden Teil eine wäßrige Lösung von Ätznatron und Na-Dithionit eingesetzt. Der pH-Wert des Nachreinigungsbades ist daher dementsprechend hoch und liegt bei etwa 12 - 13. Waschbäder, welche Na-Hydroximethansulfinat (Rongalit®c, Superlite®c) ggfs. in Mischungen mit Na-Hydroximethansulfonat oder Dithionit-Formaldehydkondensate enthalten und welche im Einzelfall auch durch Zusatz von starken organischen oder anorganischen Säuren sauer gestellt sein können, werden in den japanischen Schriften JP-A-05 272 075-A, JP-A-02 300 391, JP-A-04 146 279, JP-A-01 028 090, JP-A-02 091 285, JP-A-57 006 188, JP-A-57 066 189, JP-A-60 162 889 aufgeführt. Diese Waschsysteme werden jedoch nicht den hohen Anforderungen gerecht, die man an Nachbehandlungsmittel (NBM) hinsichtlich der Erzielung hoher Farbechtheiten bei polyesterhaltigen Textilien stellt.

Nachteile des herkömmlichen HT-AZ-Verfahrens sind

- hoher Zeit-, Wasser- und Energieeinsatz durch die pH-Wert-Umstellung von sauer nach alkalisch
- hohe Salzfrachten im Färbereiabwasser sowie Farbstoffreste, welche dieses färben
- hohe Selbstentzündlichkeitsneigung von Hydrosulfit in Substanz sowie
- eine relativ schnelle Erschöpfung des reduktiven Waschbades durch Luftoxidation.

Im Thermosol- sowie Heißdampffixierungsverfahren sind Nachteile ebenfalls in der mangelnden Luftstabilität der Waschbäder zu sehen, so daß eine exakte Dosierung des Reduktionsmittels ohne analytischen Aufwand im Betrieb nicht bewerkstelligt werden kann. Als Folge davon läßt sich eine Anschmutzung des Weißfonds durch nicht vollständig zerstörte Farbstoffreste nicht immer vermeiden.

Es bestand daher die Aufgabe, ein vereinfachtes Verfahren zur Flottenfärbung polyesterhaltiger Textilien bereitzustellen, in welchem eine aufwendige Zwischenspülung und eine separate Nachreinigung entfallen können, was eine Zeit-, Wasser- und Energieeinsparung bedeutet. Weiter sollten ungefärbte, salzarme Abwässer resultieren und die in der Färbeflotte einzusetzenden NBM pro-blemlos handhabbar, ergiebig in ihrer Wirkung und einfach zu dosieren sein sowie gute Reinigungswirkung besitzen.

Demgemäß konnte ein Verfahren zur reduktiven Nachreinigung von gefärbten, polyesterhaltigen Textilien bereitgestellt werden, das dadurch gekennzeichnet ist, daß man der sauren Färbeflotte als Nachbehandlungsmittel eine Mischung zusetzt, welche als Komponenten enthält

a) mindestens eine Verbindung der Formel (I)

$$A_m[(CR^1R^2)_mSO_2M]_{p,q} \qquad (I)$$

worin bedeuten

| | |
|---|---|
| A | $NR^3_{3-q}$ oder $OR^4_{2-p}$ |
| $R^1,R^2,R^4$ | Wasserstoff, $C_1$-$C_6$-Alkyl |
| $R^3$ | identische oder verschiedene Reste ausgewählt aus der Gruppe Wasserstoff, $C_1$-$C_{20}$-Alkyl, $C_3$-$C_8$-Cycloalkyl ggf. substituiert durch ein bis drei $C_1$-$C_4$-Alkylreste |
| M | Äquivalent eines Ammonium-, Alkali- oder Erdalkaliions |
| m | 0 oder 1 |
| p | 1 oder 2 |
| q | 1, 2 oder 3 |

wobei mindestens einer der Reste $R^1$, $R^2$, $R^4$ ein $C_1$-$C_6$-Alkyl ist, wenn A für $OR^4_{2-p}$ steht, und wobei für den Fall, daß m gleich 0 ist:

p, q den Wert 2 annimmt

und
die Verbindung der Formel (I) in Mischung mit einer Menge an säurebindender Substanz verwendet wird, welche ausreicht, um den pH-Wert der Färbeflotte um 1 bis 3 Einheiten zu erhöhen,

b) gegebenenfalls mindestens eine Verbindung der Formel (II)

$$A[(CR^1R^2)SO_3M]_{p,q} \qquad (II)$$

worin A, $R^1$, $R^2$, $R^3$, $R^4$, M, p und q dieselbe allgemeine Bedeutung wie in Formel (I) besitzen, wobei die Auswahl dieser Variablen im konkreten Einzelfall für die Verbindungen der Formeln (I) und (II) nicht gleich sein muß

c) und gegebenenfalls weitere Zusatzstoffe.

Im Falle der verfahrensgemäßen Verwendung von Mischungen, welche Ammonium-, Alkali- oder Erdalkalidithionite enthalten - hier ist m in Formel (I) gleich 0 - werden als säurebindende Substanzen bevorzugt Ammonium-, Alkali- oder Erdalkalihydrogencarbonat, -carbonat, -oxid, -hydroxid, -hydrogensulfit, -sulfit, -hydrogenphosphat oder -phosphat eingesetzt, wobei die Verwendung der Alkali- und Erdalkalihydrogencarbonate und -carbonate besonders bevorzugt ist. Üblicherweise wird man das unter der Bezeichnung "Hydrosulfit" kommerziell verfügbare Natriumdithionit einsetzen. Dieses hat in der Regel eine technische Reinheit von mehr als 80 %. Seine Verwendung wird in dem hier beschriebenen Nachreinigungsverfahren aus Kostengründen bevorzugt. Spezielle Anforderungen können jedoch den Einsatz anderer Alkali- sowie Erdalkalioder Ammoniumdithionite oder deren Mischungen rechtfertigen.

Als Ammoniumdithionite lassen sich nicht nur $NH_4^{\oplus}$- sondern beispielsweise auch $NL_4^{\oplus}$-Verbindungen einsetzen, worin die Substituenten L identische oder verschiedene Reste aus der Gruppe Wasserstoff oder $C_1$-$C_6$-Alkyl, in welchem nicht benachbarte CH- bzw. $CH_2$-Gruppen durch N bzw. NH oder O und $CH_3$-Gruppen durch $NH_2$ oder OH ersetzt sein können, darstellen. Beispiele hierfür sind die Mono-, Di-, Tri- und Tetraethanolammoniumverbindungen aber auch die entsprechenden Salze des Ethylendiamins, Diethylentriamins und Triethylentetramins. Möglich sind auch die Salze der Kondensationsprodukte aus Ammoniak mit Diethylenglykol wie $H_2N(C_2H_4)O(C_2H_4)OH$, $HN[(C_2H_4)O(C_2H_4)(OH]_2$ oder $N[(C_2H_4)O(C_2H_4)OH]_3$.

Als säurebindende Substanzen können neben den bereits erwähnten Stoffen auch deren Mischungen verwendet werden. Wichtig ist lediglich eine "Pufferung" der sauren Färbeflotte um etwa 1 - 3 pH-Wert-Einheiten, um die Reduktion überschüssig vorhandenen Farbstoffs durch das Dithionit sicherzustellen.

Im Normalfall ist dabei die gewünschte pH-Wert-Änderung durch Einsatz von Dithioniten und säurebindender Substanz bei einem Gewichtsverhältnis von 3 : 1 bis 1 : 1 gewährleistet, weshalb dieses Mischungsverhältnis auch bevorzugt eingesetzt wird.

Je nach den speziellen Erfordernissen kann es auch sinnvoll sein, Salze von Komplexbildnern wie z.B. Nitrilotriessigsäure (NTA, $N(CH_2COOH)_3$, Ethylendiamintetraessigsäure (EDTA, $[CH_2N(CH_2COOH)_2]_2$), Diethylentriaminpentaessigsäure (DTPA, $HOOCCH_2N[(CH_2)_2N(CH_2COOH)_2]_2$), Hydroxyethylethylendiamintriessigsäure (HEDTA, $HO(CH_2)_2N(CH_2COOH)(CH_2)_2N(CH_2COOH_2)$, Propylendiamintetraessigsäure (PDTA, $(HOOCCH_2)_2N(CH_2)_3N(CH_2COOH)_2$) oder β-Alanindiessigsäure ($(HOOCCH_2)_2N(CH_2)_2COOH$) als Puffer zuzugeben. Diese werden z. B. unter dem Markennamen Trilon® vertrieben. Auch Mischungen dieser Komplexbildner untereinander sowie mit anderen säurebindenden Stoffen sind möglich.

Werden zusätzlich noch Tenside oder Dispergiermittel in der Nachbehandlung eingesetzt, so kann - wenn nötig - eine Maskierung von eventuell störenden Schwermetallionen oder auch eine Herabsetzung der Wasserhärte durch den Einsatz von solchen Komplexbildnern erfolgen.

Weiter können dem NBM auf Basis von Dithioniten und säure-bindenden Substanzen auch Sulfonate entsprechend Formel (II) zugegeben werden. Verbindungen wie etwa $HOCH(CH_3)SO_3M$ bis $HOCH(C_6H_{13})SO_3M$ lassen sich beispielsweise durch Addition von Bisulfit $MHSO_3$, wobei M vorzugsweise ein Alkalimetall wie Natrium ist, an Acetaldehyd bzw. Önanthaldehyd, $C_6H_{13}CHO$, herstellen. Auch bei der Umsetzung von Aldehyden mit Dithioniten entstehen, in Mischung mit den entsprechenden Sulfinaten und abhängig von der Reaktionsführung und den Molverhältnissen, diese Sulfonate.

Durch Reaktion von Hydroxyalkansulfonaten mit Ammoniak in unter-schiedlichen Mengenverhältnissen werden die Amino-, Imino- und Nitrilosulfonate erhalten. Ausgehend von den entsprechenden Hydroxyalkansulfonaten sind dies beispielsweise die Verbindungen $HN(CH_2SO_3M)_2$, $N(CH_2SO_3M)_3$, $H_2NCH(CH_3)SO_3M$, $HN(CH(CH_3)SO_3M)_2$, $N(CH(CH_3)SO_3M)_3$ bis $H_2NCH(C_6H_{13})SO_3M$, $HN(CH(C_6H_{13})SO_3M)_2$, und $N(CH(C_6H_{13})SO_3M)_3$ teilweise auch noch in Mischung mit den entsprechenden Ausgangssulfonaten. Weiter kann auch $H_2NCH_2SO_3M$ eingesetzt werden.

Setzt man Hydroxyalkansulfonate z. B. mit primären oder sekundä-ren Aminen um, so lassen sich die entsprechenden N-substituierten Amino- oder Aminoalkansulfonate gewinnen. Hier sind bevorzugt Amine mit $C_1$-$C_{20}$-Alkylresten sowie $C_3$-$C_8$-Cycloalkylresten, die ggf. noch mit ein bis drei weiteren $C_1$-$C_4$-Alkylresten substituiert sein können, einzusetzen.

Bei Verwendung der primären Amine erhält man beispielsweise die N-alkylierten Aminomethansulfonate $R^3NHCH_2SO_3M$ bei der Umsetzung mit $HOCH_2SO_3M$. Zu den Resten $R^3$ zählen nicht nur lineare $C_1$-$C_{20}$-Alkylketten wie Methyl, Ethyl, Propyl, Butyl, Pentyl, Hexyl usw. bis zu Eikosyl, sondern auch verzweigte $C_1$-$C_{20}$-Reste wie etwa die verschiedenen Methylbutyle, Ethylbutyle, Methylpentyle, Ethylpentyle, Methylhexyle, Ethylhexyle, Methylheptyle, Ethylhexyle usw. Daraus resultieren Sulfonate wie $(H_3C)NHCH_2SO_3M$, $(H_5C_2)NHCH_2SO_3M$, $(H_7C_3)NHCH_2SO_3M$, $(H_9C_4)NHCH_2SO_3M$, $(H_{11}C_5)NHCH_2SO_3M$, $(H_{13}C_6)NHCH_2SO_3M$ bis zu $(H_{41}C_{20})NHCH_2SO_3M$ aber auch 1-,2-,3-Methylbutylaminomethansulfonat, 1-,2-,3-Ethylbutylaminomethansulfonat, 1-,2-,3-,4-Methylpentylaminomethansulfonat, 1-,2-,3-,4-Ethylpentylaminomethansulfonat, 1-,2-,3-,4-,5-Methylhexylaminomethansulfonat, 1-,2-,3-,4-,5-Ethylhexylaminomethansulfonat, 1-,2-,3-,4-,5-,6-Methylheptylaminomethansulfonat, 1-,2-,3-,4-,5-,6-Ethylheptylaminomethansulfonat usw. Im Falle der, ggf. durch $C_1$-$C_4$-Alkylreste substituierten, $C_3$-$C_8$-Cycloalkylreste ergeben sich, wie exemplarisch für den Cyclohexyl-Rest aufgeführt, die nachfolgenden Sulfonate:

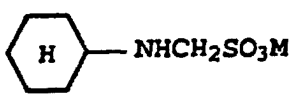

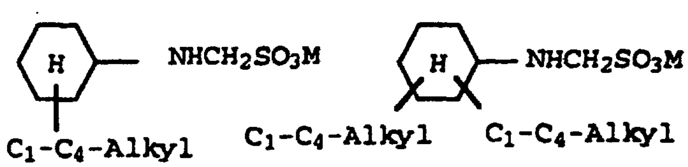

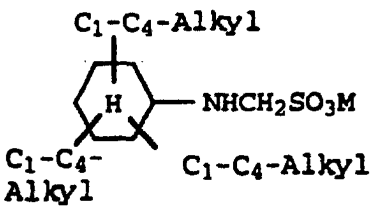

Entsprechende Iminoalkansulfonate lassen sich bei Ersatz des Aminowasserstoffs durch eine weitere z. B. Alkansulfonat-Gruppierung herstellen. Entsprechend sind daher auch in der Synthese die Mengen an Alkylamin und Hydroxyalkansulfonat zu wählen, wobei letzteres auch aus einer Mischung verschiedener Hydroxyalkansulfonate bestehen kann. Im Falle der symmetrisch durch Alkansulfonatreste substituierten Amine erhält man also Aminoalkansulfonate wie z. B. $(H_3C)N(CH_2SO_3M)_2$, sofern von Methylamin und Hydroxymethansulfonat im Molverhältnis 1 : 2 ausgegangen wird. Analog lassen sich auch alle schon zuvor aufgeführten Aminomethansulfonate in die entsprechenden Iminomethansulfonate überführen. Bei Verwendung der sekundären Amine wird die Vielfalt der Verbindungen nicht nur, wie bereits angedeutet, durch die Möglichkeit des Einsatzes von Mischungen verschiedener Hydroxyalkansulfonate sondern zusätzlich noch durch die Möglichkeit der Wahl unterschiedlicher Alkylreste $R^3$ am Ausgangsamin bestimmt. Wegen der Vielzahl der unterschiedlichen, durch Umsetzung von $R^3{}_2NH$ mit Hydroxyalkansulfonaten (oder Mischungen daraus) zugänglichen N,N-disubstituierten Aminoalkansulfonate wird jedoch auf eine detaillierte Aufführung nicht näher eingegangen.

Weiterhin sei angemerkt, daß bei der Umsetzung von Ammoniak oder primären Aminen $R^3NH_2$ mit verschie-

denen Hydroxyalkansulfonaten auch gezielt "molekular gemischte" Kitriloalkan- bzw. Iminoalkansulfonate hergestellt werden können.

Für zwei Hydroxyalkansulfonate - im folgenden als $HOAlkSO_3(1)$ und $HOAlkSO_3(2)$ abgekürzt - sei die Zahl und Art der gewünschten Verbindungen durch die Schemata (a) und (b) verdeutlicht:

(a) $$NH_3 + HOAlkSO_3(1) \rightarrow H_2NAlkSO_3(1) + H_2O$$

$$H_2NAlkSO_3(1)+2HOAlkSO_3(2) \rightarrow N(AlkSO_3(1))(AlkSO_3(2))_2 +2H_2O$$

oder

$$NH_3 + HOAlkSO_3(2) \rightarrow H_2NAlkSO_3(2) + H_2O$$

$$H_2NAlkSO_3(2)+2HOAlkSO_3(1) \rightarrow N(AlkSO_3(2))(AlkSO_3(1))_2 +2H_2O$$

(b) $$R^3NH_2 + HOAlkSO_3(1) + HOAlkSO_3(2) \rightarrow R^3N(AlkSO_3(1))(AlkSO_3(2)) + 2H_2O$$

Selbstverständlich sollen auch solche "intramolekular gemischten" Sulfonate erfindungsgemäß eingeschlossen sein.

Vorzugsweise finden die Natriumsulfonate und besonders bevorzugt die Verbindungen $H_2NCH_2SO_3Na$, $HN(CH_2SO_3Na)_2$, $N(CH_2SO_3Na)_3$, $HOCH(CH_3)SO_3Na$, $H_2NCH(CH_3)SO_3Na$, $HN(CH(CH_3)SO_3Na)_2$, $N(CH(CH_3)SO_3Na)_3$ und Natriumsalze der 1-,2-,3-,4-,5-Ethylhexylaminomethansulfonsäure und-ethansulfonsäure Verwendung.

Das bevorzugte Molverhältnis von Dithionit zu Sulfonat liegt bei 20 : 1 bis 1 : 20, insbesondere bei 10 : 1 bis 1 : 10.

Die erfindungsgemäß einzusetzenden Hydroxy-, Amino-, Imino- und Nitriloalkansulfinate, hier ist m in Formel (I) gleich 1, lassen sich formal aus den bereits oben aufgeführten Sulfonaten durch Ersatz der $SO_3M$- durch eine $SO_2M$-Gruppe ableiten. So resultieren beispielsweise Verbindungen wie $HOCH(CH_3)SO_2M$ bis zu $HOCH(C_6H_{13})SO_2M$ aber auch $H_2NCH_2SO_2M$, $HN(CH_2SO_2M)_2$, $N(CH_2SO_2M)_3$, $H_2NCH(CH_3)SO_2M$, $HN(CH(CH_3)SO_2M)_2$, $N(CH(CH_3)SO_2M)_3$ bis $H_2NCH(C_6H_{13})SO_2M$, $HN(CH(C_6H_{13})SO_2M)_2$, $N(CH(C_6H_{13})SO_2M)_3$. Durch Ersatz des Aminowasserstoffs mit einem oder ggf. zwei Resten $R^3$ lassen sich die N-substituierten Sulfinate herstellen. Wie im Falle der bereits erwähnten Sulfonate kommen für $R^3$ bevorzugt lineare oder verzweigte $C_1$-$C_{20}$-Alkyl- sowie ggf. mit ein bis drei $C_1$-$C_4$-Alkylresten substituierte $C_3$-$C_8$-cycloaliphatische Reste in Frage.

Am Beispiel des Aminomethansulfinats, $H_2NCH_2SO_2M$, ausgeführt, ergeben sich die N-substituierten Verbindungen wie Methyl-, Ethylbis zu Eikosylaminomethansulfinat aber auch 1-,2-,3-Methylbutyl-, 1-,2-,3-Ethylbutyl-, 1-,2-,3-,4-Methylpentyl-, 1-,2-,3-,4-Ethylpentyl-, 1-,2-,3-,4-,5-Methylhexyl-, 1-,2-,3-,4-,5-Ethylhexylaminomethansulfinat usw. Analog wie bereits bei den Sulfonaten aufgeführt lassen sich beispielsweise die ggf. am Cyclohexanring substituierten Cyclohexylaminomethansulfinate ableiten.

Entsprechend zu den erwähnten Iminoalkansulfonaten lassen sich natürlich auch durch zweimaligen Ersatz der Wasserstoffe im primären Amin $R^3NH_2$ durch Alkansulfinatreste die analogen Iminoalkansulfinate herstellen, wobei im Falle der Synthese aus dem Amin $R^3NH_2$ und einem Hydroxyalkansulfinat oder einer Mischung von Sulfinaten je nach Mengenverhältnis auch eine Imino-/Aminoalkansulfinatmischung resultieren kann. Deren Verwendung im erfindungsgemäßen Verfahren ist ebenfalls möglich. Im Hinblick auf die N,N-disubstituierten Aminoalkansulfinate gilt sinngemäß das bereits weiter oben zu den Sulfonaten Gesagte.

Werden NBM auf Basis der Sulfinate zusammen mit mindestens einem Sulfonat der Formel (II) eingesetzt, so ist ein Molverhältnis von Sulfinat zu Sulfonat von 20 : 1 bis 1 : 20, insbesondere ein Molverhältnis von 10 : 1 bis 1 : 10 bevorzugt. Weiter können auch hier säurebindende Substanzen beigemengt werden.

Ein leichter Zugang zu Mischungen von Alkansulfinaten mit Alkansulfonaten z. B. im Molverhältnis 1 : 1 ist

durch Reaktion der Aldehyde $R^1$-CHO bzw. Ketone $R^1$-CO-$R^2$ mit Dithioniten im Verhältnis Carbonylverbindung zu Dithionit gleich 2 : 1 möglich. So erhält man durch Umsetzung von Natriumdithionit mit Acetaldehyd, Propionaldehyd, Butyraldehyd, n-Valeraldehyd, Capronaldehyd oder Önanthaldehyd Mischungen von Sulfinaten und Sulfonaten, die den Formeln $HOCH(CH_3)SO_2M$ bzw. $HOCH(CH_3)SO_3M$, $HOCH(C_2H_5)SO_2M$ bzw. $HOCH(C_2H_5)SO_3M$, $HOCH(C_3H_7)SO_2M$ bzw. $HOCH(C_3H_7)SO_3M$, $HOCH(C_4H_9)SO_2M$ bzw. $HOCH(C_4H_9)SO_3M$, $HOCH(C_5H_{11})SO_2M$ bzw. $HOCH(C_5H_{11})SO_3M$ und $HOCH(C_6H_{13})SO_2M$ bzw. $HOCH(C_6H_{13})SO_3M$ entsprechen, wobei M einem Äquivalent eines Ammonium-, Alkali- oder Erdalkaliions entspricht, bzw. - wie im Falle der Verwendung von Natriumdithionit - natürlich Na. Auch die Verwendung von Aldehyden mit verzweigten Ketten ist möglich, so daß Sulfinate bzw. Sulfonate mit den $C_6$-Resten $R^1$ wie 1-,2-,3-,4-Methylpentyl und 1-,2-Ethylbutyl, den $C_5$-Resten 1-,2-,3-Methylbutyl (Isoamyl) und 1-Ethylpropyl, und den $C_4$-Resten 1-,2-Methylpropyl (Isobutyl) resultieren.

Geht man von Aldehyden zu den Ketonen als Umsetzungspartner für Dithionit über, so ergeben sich die symmetrischen Verbindungen beginnend mit Methylmethylketon, dem Aceton, bis zum Hexylhexylketon, $(H_{13}C_6)CO(C_6H_{13})$ sowie die größere Gruppe der unsymmetrischen Ketone beginnend mit Methylethylketon bis zum Methylhexylketon über Ethylpropylketon bis Ethylhexylketon usw. bis letztlich zum Pentylhexylketon. Daraus leiten sich die entsprechenden Sulfinate/Sulfonate $HOC(CH_3)_2SO_2M/HOC(CH_3)_2SO_3M$ bis $HOC(C_6H_{13})_2SO_2M/HOC(C_6H_{13})_2SO_3M$ sowie die unsymmetrisch substituierten Verbindungen $HOC(CH_3)(C_2H_5)SO_2M/HOC(CH_3)(C_2H_5)SO_3M$ bis $HOC(CH_3)(C_6H_{13})SO_2M/HOC(CH_3)(C_6H_{13})SO_3M$, $HOC(C_2H_5)(C_3H_7)SO_2M/HOC(C_2H_5)(C_3H_7)SO_3M$ bis $HOC(C_2H_5)(C_6H_{13})SO_2M/HOC(C_2H_5)(C_6H_{13})SO_2M$ usw. bis zu $HOC(C_5H_{11})(C_6H_{13})SO_2M/HOC(C_5H_{11})(C_6H_{13})SO_3M$ ab. Auch hier können natürlich, wie bei den oben erwähnten Umsetzungen von Aldehyden, die entsprechend verzweigten aliphatischen Reste auftreten.

Bringt man die ggf. substituierten Hydroxymethansulfinate und Hydroxymethansulfonate mit Ammoniak zur Reaktion, so ergeben sich je nach Verhältnis der Reaktanden mono-, di- oder trisubstituierte Amine der allgemeinen Form $H_2NCR^1R^2SO_2M$ bzw. $H_2NCR^1R^2SO_3M$, $HN(CR^1R^2SO_2M)_2$ bzw. $HN(CR^1R^2SO_3M)_2$ und $N(CR^1R^2SO_2M)_3$ bzw. $N(CR^1R^2SO_3M)_3$. Eine Mischung von Hydroxymethansulfinat. $HOCH_2SO_2M$, mit Hydroxymethansulfonat, $HOCH_2SO_3M$, führt in der Reaktion mit Ammoniak und abhängig vom Sulfinat/Sulfonat: Ammoniak-Verhältnis zu den entsprechenden Aminomethansulfinat/-sulfonat-, Iminomethansulfinat/-sulfonat-, Nitrilomethansulfinat/-sulfonat-Mischungen $H_2NCH_2SO_2M/H_2NCH_2SO_3M$, $HN(CH_2SO_2M)_2/HN(CH_2SO_3M)_2$, bzw. $N(CH_2SO_2M)_3/N(CH_2SO_3M)_3$. Bei Verwendung symmetrisch oder unsymmetrisch substituierter Hydroxyalkylsulfinate/-sulfonate, wie sie beispielhaft weiter oben bereits aufgeführt wurden, ergeben sich etwa für die Ausgangssubstanzen $HOC(CH_3)_2SO_2M/HOC(CH_3)_2SO_3M$ die folgenden Verbindungen $H_2NC(CH_3)_2SO_2M/H_2NC(CH_3)_2SO_3M$, $HN(C(CH_3)_2SO_2M)_2/HN(C(CH_3)_2SO_3M)_2$, $N(C(CH_3)_2SO_2M)_3/N(C(CH_3)_2SO_3M)_3$ oder etwa für die Ausgangssubstanzen $HOC(CH_3)(C_2H_5)SO_2M/HOC(CH_3)(C_2H_5)SO_3M$ die Verbindungen $H_2NC(CH_3)(C_2H_5)SO_2M/H_2NC(CH_3)(C_2H_5)SO_3M$, $HN(C(CH_3)(C_2H_5)SO_2M)_2/HN(C(CH_3)(C_2H_5)SO_3M)_2$ und $M(C(CH_3)(C_2H_5)SO_2M)_3/N(C(CH_3)(C_2H_5)SO_3M)_3$.

Gewünschtenfalls lassen sich andere Mischungen von Sulfinaten ggf. zusammen mit Mischungen von Sulfonaten einsetzen. Mischungen von Sulfinaten mit Sulfonaten lassen sich auch einfach durch unvollständige Umsetzungen herstellen. Bei Verwendung einer Mischung von $HOCH(CH_3)SO_2M/HOCH(CH_3)SO_3M$ und Einsatz einer Ammoniakmenge, welche nicht ausreicht, um die entsprechenden Nitriloverbindungen zu erhalten, liegen nach beendeter Umsetzung neben dem Reaktionsprodukt $N(CH(CH_3)SO_2M)_3/N(CH(CH_3)SO_3M)_3$ auch noch die nicht abreagierten Ausgangskomponenten $HOCH(CH_3)SO_2M$ und $HOCH(CH_3)SO_3M$ vor. Sinngemäß läßt sich so auch ein beliebiges Verhältnis von z. B. Amino- oder Imino- zu den Nitrilosulfinaten/-sulfonaten einstellen. Noch komplexere Mischungen sind erhältlich durch Einsatz gemischter Hydroxyalkylsulfinat/-sulfonat-Ausgangskomponenten. So lassen sich die Sulfinate $HO(CH_3)_2SO_2M$ und $HOCH(CH_3)SO_2M$ in Mischung mit den Sulfonaten $HO(CH_3)_2SO_3M$ und $HOCH(CH_3)SO_3M$ durch Umsetzung einer Aceton/Acetaldehyd-Mischung mit Dithionit erhalten und entsprechend dem oben bereits Ausgeführten mit einem Unterschuß an Ammoniak zu den Nitrilofolgeprodukten in Mischung mit den Ausgangskomponenten umsetzen.

Werden die Hydroxyalkansulfinate, falls gewünscht in Mischung mit den Sulfonaten, nicht mit Ammoniak sondern mit einem primären oder sekundären Amin $R^3HH_2$ bzw. $R_2{}^3NH$ umgesetzt, so gelangt man zu den entsprechenden N-alkylierten Produkten ggf. in Mischung mit ihren zugrundeliegenden Hydroxyalkansulfinaten/-sulfonaten. Im Falle der primären Amine $R^3NH_2$ sind dabei jedoch maximal N-alkylierte Iminoalkan-, im Falle der sekundären Amine $R_2{}^3NH$ nur N-alkylierte Aminoalkansulfinate/-sulfonate erhältlich.

Für NBM auf Basis von Dithionit wurden bereits weiter oben exemplarisch am "Grundkörper" $HOCH_2SO_3M$ verschiedene N-alkylierte Derivate mit unterschiedlichen Resten $R^3$ aufgezeigt. Natürlich lassen sich auch alle bereits genannten Hydroxysulfinate/-sulfonate, die durch Reaktion von Aldehyden, Ketonen oder deren Mischungen mit Dithioniten erhalten werden können, mit diesen Aminen zu den N-substituierten Amino-bzw. Iminosulfinaten/-sulfonaten umsetzen. Aus der Vielzahl der möglichen und erfindungsgemäß zu verwendenden Sulfinate/Sulfonate sollen daher, ohne daß eine Einschränkung im Hinblick auf die Erfindung erfolgen soll, nur exemplarisch für eine Hydroxyethansulfinat/-sulfonat-Mischung die verschiedenen Reaktionsprodukte aus der Umsetzung mit z. B. 2-Ethylhexylamin ($R^3$=2-Ethylhexyl) abgeleitet werden.

Setzt man das Amin mit dem Sulfinat und Sulfonat im molaren Verhältnis 2 : 1 : 1 um, so erhält man eine äquimolare Mischung von 2-Ethylhexylaminoethansulfinat und 2-Ethylhexylaminoethansulfonat, $(2\text{-}C_2H_5\text{-}C_6H_{12})NHCH(CH_3)SO_2M$ und $(2\text{-}C_2H_5\text{-}C_6H_{12})NHCH(CH_3)SO_3M$.

Bei einer Umsetzung des Amins mit dem Sulfinat/Sulfonat im molaren Verhältnis 1 : 1: 1 wird eine äquimolare Mischung des 2-Ethylhexyliminoethansulfinats und -sulfonats, $(2\text{-}C_2H_5\text{-}C_6H_{12})N(CH(CH_3)SO_2M)_2$ und $(2\text{-}C_2H_5\text{-}C_6H_{12})N(CH(CH_3)SO_3M)_2$, er-halten. Mischungen des Aminoethansulfinats und -sulfonats mit dem Ausgangssulfinat/-sulfonat erhält man, wenn der molare Anteil des Amins noch kleiner gewählt wird. All diese Mischungen sind im erfindungsgemäßen Verfahren ebenfalls anwendbar.

Vorzugsweise werden die Natriumsalze, besonders bevorzugt die Sulfinate $HOCH(CH_3)SO_2Na$, $H_2NCH_2SO_2Na$, $HN(CH_2SO_2Na)_2$, $N(CH_2SO_2Na)_3$, $H_2NCHCH_3SO_2Na$, $HN(CH(CH_3)SO_2Na)_2$, $N(CH(CH_3)SO_2Na)_3$, Natrium-1-,2-,3-,4-,5-Ethylhexylaminomethansulfinat sowie Natrium-1-,2-,3-,4-,5-Ethylhexylaminoethansulfinat eingesetzt, wobei letztere Verbindungen durch Umsetzung des 1-,2-,3-,4- oder 5-Ethylhexylamins mit dem Natrium-Hydroxymethansulfinat bzw. Natrium-Hydroxyethansulfinat in äquimolaren Verhältnissen hergestellt werden können. Ggf. können diese Verbindungen in Mischung mit den Sulfonaten, $HOCH(CH_3)SO_3Na$, $H_2NCH_2SO_3Na$, $HN(CH_2SO_3Na)_2$, $N(CH_2SO_3Na)_3$, $H_2N\text{-}CH(CH_3)SO_3Na$, $HN(CH(CH_3)SO_3Na)_2$, $N(CH(CH_3)SO_3Na)_3$, Natrium-1-,2-,3-,4-,5-Ethylhexylaminomethansulfonat und -ethansulfonat verwendet werden.

Natürlich können erfindungsgemäß auch, wie bereits oben für die Sulfonate in den Schemata (a) und (b) exemplarisch ausgeführt, "molekular gemischte" Sulfinate/Sulfonate eingesetzt werden. Durch Schema (c) soll beispielhaft die Art der durch gezielte Umsetzung von Ammoniak mit einem Hydroxyalkansulfinat und dem entsprechendem Sulfonat - im folgenden als $HOAlkSO_2$ und $HOAlkSO_3$ bezeichnet - erhältlichen Verbindungen veranschaulicht werden:

$$\text{(c)}\ NH_3 + HOAlkSO_2 \rightarrow H_2NAlkSO_2 + H_2O$$

$$H_2NAlkSO_2 + 2HOAlkSO_3 \rightarrow N(AlkSO_2)(AlkSO_3)_2 + 2H_2O$$

oder

$$NH_3 + HOAlkSO_3 \rightarrow H_2NAlkSO_3 + H_2O$$

$$H_2NAlkSO_3 + 2HOAlkSO_2 \rightarrow N(AlkSO_3)(AlkSO_2)_2 + 2H_2O$$

Es sei noch angemerkt, daß natürlich aufgrund unvollständiger Umsetzung oder eines Überschusses an Ammoniak neben den in Schema (c) aufgeführten Produkten zusätzlich noch Reste der Ausgangsoder entsprechende Iminoverbindungen zugegen sein können. Sinngemäß gelten diese Überlegungen auch bei Verwendung von primären Aminen anstelle des Ammoniaks.

Neben den bereits angesprochenen Komponenten Dithionit/säurebindende Substanz bzw. Sulfinat ggf. in Mischung mit Sulfonat können die verfahrensgemäß einzusetzenden NBM weitere Zusatzstoffe enthalten. So kann man mindestens ein Hydroxyketon oder ein sich aus mehreren gleichen oder verschiedenen Hydroxyketonen ableitendes oligo- oder polymeres Produkt zugeben. Der Anteil dieser Verbindungen an der Gesamtmischung liegt vorzugsweise bei 5 - 60 Gew.-%.

Zu diesen Substanzen zählen z. B. Acetoin $((H_3C)CH(OH)CO(CH_3))$, Hydroxyaceton, cyclische Verbindungen wie

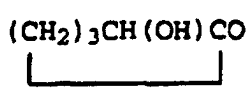
Glutaroin

oder

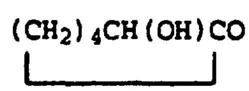
Adipoin

aber auch Reduktinsäure,

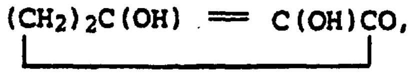

und Ascorbinsäure.

Weiterhin können erfindungsgemäß als Monosaccharide Aldosen wie Erythrose, Threose, Ribose, Xylose, Arabinose, Glucose, Mannose, Galaktose oder Ketosen wie Fructose oder Sorbose eingesetzt werden.

Unter den oligo- oder polymeren Hydroxyketonen sind bevorzugt die entsprechenden Oligo- oder Polysaccharide zu verstehen. Beispielsweise sind hier zu nennen die Disaccharide Saccharose, Laktose, Maltose, Cellobiose oder Trisaccharide wie Raffinose und Maltotriose. Weiter können auch Stärken, welche chemisch gesehen Polysaccharide überwiegend auf Basis der monomeren Glucose-Einheit darstellen, sowie deren partielle Hydrolyseprodukte eingesetzt werden. Beispielsweise erhält man durch Hydrolyse der Stärkebestandteile Amylose und Amylopektin letztlich Maltose und Glucose. Für den verfahrensgemäßen Einsatz der NBM kann daher durch Beimischung von hydrolysierbaren Oligo- und Polysacchariden eine zusätzliche säurebindende Wirkung erzielt werden. Bevorzugt werden dem NBM Hydroxyaceton, Acetoin, Glutaroin oder Adipoin, besonders bevorzugt Glucose, Fructose oder Saccharose alleine oder auch in Mischung zugesetzt.

Weiter kann man dem NBM mindestens ein Dispergiermittel oder Tensid oder Mischungen daraus beimischen. Der Anteil an der Gesamtmischung beträgt dabei bevorzugt 2 - 50 Gew.-%. Diese Hilfsmittel können sowohl kationische, anionische, nichtionische als auch zwitterionische Verbindungen sein. Erfindungsgemaß lassen sich dabei als Dispergiermittel einsetzen - ohne daß durch die Reihenfolge der nachfolgenden Aufführung eine Zuordnung zu einer dieser Klassen beabsichtigt ist - Polycarboxylate und Copolymerisate, die z. B. unter den Markennamen Sokalan® oder Elvacite®, oder auch sogenannte "Hyperdispersants", die unter dem Namen Solsperse®, vertrieben werden, weiterhin Kondensate auf Basis aromatischer oder alkylaromatischer Sulfonate, die unter den Markennamen Tamol® und Nekal® sowie Supragil® und Rhodacar® erhältlich sind.

Als Tenside sind beispielsweise zu verwenden:

Alkoxylierungsprodukte, die basierend auf aliphatischen oder alkylaromatischen Hydroxy-, Amin- und Aminohydroxyverbindungen unter den Markennamen Synperonic® und Ukanil®, Dehypon®, Neopol®-Ethoxylate, Emulan®, Lutensol®, Plurafac® und Pluronic® oder Elfapur® kommerziell erhältlich sind,

Polyalkylenglykole, bekannt unter den Markennamen Pluriol® und Antarox®,

aliphatische und alkylaromatische Ein- und Mehrfachsulfonate mit den Markennamen Lutensit®, Rhodacar®, Rhodapon® und Teepol®,

Säureester und -Amide wie z. B. Sulfobernsteinsäureester der Marke Elfanol®,

Phosporsaurepartialester, die unter den Namen Rhodafac® oder auch Marlophor® vertrieben werden,

Fettsaurepartialglyceride sowie Fettsäurealkanolamide, die unter den Markennamen Luwitor® bzw. Marlamid® zu erhalten sind,

sowie die unter den Namen Plantaren® und Glucopon® angebotenen Tenside.

Ferner können besonders vorteilhaft noch Tenside mit Betain- oder Sultain-Gruppierungen Einsatz finden, welche innere Salze aus quartärnären Ammonium- und Carboxylat-(Betain) bzw. Sulfonat (Sultain)-Ion sind und z. B. unter den Markennamen Mackam® erhältlich sind. Weiterhin können auch kationische Tenside auf Basis quartärnärer Ammoniumverbindungen und Aminoxide Einsatz finden, wie sie etwa unter den Namen Alkaquat® und Rhodaquat® sowie Mackalene®, Mackernium®, Mackpro® und Mackamine® angeboten werden.

Als weiteren Zusatzstoff kann man dem NBM noch mindestens ein Ammonium-, Alkali- oder Erdalkalisulfit-, -hydrogensulfit (-bisulfit) oder -disulfit zumischen. Der Anteil an der Gesamtmischung beträgt bevorzugt 5 - 30 Gew.-%. Vorteilhaft setzt man dabei die Alkalisalze, insbesondere $Na_2SO_3$, $NaHSO_3$ oder $Na_2S_2O_5$ zu.

Falls nötig, können ferner noch Korrosionsinhibitoren beigefügt werden. Die Mengen liegen dabei - abhängig natürlich von der Art des Inhibitors - im Bereich von 1 ppm bis 1 Gew.-% bezogen auf die Gesamtmischung.

Bevorzugt wird eine Menge von 5 ppm bis 0,5 Gew.-%, besonders bevorzugt ein Anteil von 10 ppm bis 0,1 Gew.-% jeweils wieder im Bezug zur Gesamtmischung. In Betracht kommen dabei Substanzen aus den nachfolgenden Gruppen:

(a) Umsetzungsprodukte aus gesättigten oder ungesättigten aliphatischen Carbonsäuren mit 3 bis 30 C-Atomen und aliphatischen Oligoaminen mit 2 bis 8 N-Atomen, welche zusätzlich Hydroxylgruppen tragen können, oder Dialkanolaminen oder Derivate solcher Umsetzungsprodukte,

(b) aliphatische Sulfoniumsalze, welche durch zusätzliche hydrophile Gruppen substituiert sein können,

(c) aliphatische oder aromatische Monocarbonsäuren und/oder Dicarbonsäuren mit 3 bis 16 C-Atomen oder deren wasserlöslichen Salzen,

(d) Triazole oder Derivate hiervon,

(e) Imidazole oder Derivate hiervon,

(f) Thiazole oder Derivate hiervon,

(g) ungesättigte aliphatische Alkohole mit 3 bis 6 C-Atomen.

(h) Alkenylbernsteinsäure, deren wasserlösliche Salze oder Derivate solcher Alkenylbernsteinsäuren,

(j) Polymaleinsäuren oder deren wasserlösliche Salze,

(k) $\alpha$-Olefin-Maleinsäureanhydrid-Copolymere,

(l) Sulfamidocarbonsäuren oder deren wasserlösliche Salze und/ oder

(m) Ammoniumsalze von Sulfonsäuren.

Die Substanzen der Gruppe (a) und ihre Herstellung sind beispielsweise aus EP-A 034 726, DE-A 3 109 826, DE-A 3 109 827, EP-A 103 737 und DE-A 195 202 69 bekannt.
Beispiele für Verbindungen der Gruppe (b) sowie Verfahren zu deren Herstellung werden in den Schriften JP-B 1972/10202, DE-A 1 806 653 und DE-A 2 208 894 beschrieben.
Als Inhibitoren der Gruppe (c) kommen vor allem aliphatische Monocarbonsäuren mit 5 bis 12 C-Atomen und/ oder deren Natriumund Kaliumsalze sowie aliphatische Dicarbonsäuren mit 4 bis 12 C-Atomen sowie deren Mono- oder Dinatrium- bzw. -kaliumsalze in Frage. Weiterhin fallen unter diese Gruppe aber auch aromatische Carbonsäuren wie Benzoe-, Methylbenzoe-, Phthal- oder Terephthalsäure sowie deren Natrium- und Kalium- aber auch Ammoniumsalze, welche z. B. auf Piperazin oder Morpholin basieren.
Als Triazole (d), kommen insbesondere Kohlenwasserstofftriazole, vor allem Benztriazol und Toluotriazol, in Betracht.
Als Imidazole (e) eignen sich vor allem unsubstituiertes Imidazol, alkyl- oder arylsubstituierte Imidazole wie 1-($C_1$- bis $C_4$-Alkyl)imidazole oder 1-Phenylimidazol, Aminoalkylimidazole, z.B. N-(3-Aminopropyl)imidazol, sowie quaternierte Imidazole, z.B. mit Dimethylsulfat quaterniertes N-Vinylimidazol, letztere sind in der deutschen Patentanmeldung 196 05 509 als Buntmetall-Korrosionsinhibitoren beschrieben.
Als Thiazole (f) kommen insbesondere Kohlenwasserstoffthiazole, z.B. Benzothiazol, in Betracht.
Ein typischer Vertreter eines ungesättigten Alkohols (g) ist Propargylalkohol.
Bei den Alkenylbernsteinsäuren (h) bzw. deren Derivaten sind Ammoniumsalze von Alkenylbernsteinsäurehalbamiden, wie sie in der DE-A 41 03 262 beschrieben sind, besonders hervorzuheben. Als Alkenylrest ist hierbei insbesondere ein Polyisobutylrest von Interesse.
Geeignete Polymaleinsäuren (j) sind beispielsweise in der EP-A 065 191 beschrieben.
Typische $\alpha$-Olefin-Maleinsäure-Copolymere (k) liegen teilweise oder ganz zu den Dicarbonsäure-Strukturen geöffnet vor und sind meist mit Aminen zu Amiden oder Imiden derivatisiert. Als $\alpha$-Olefine kommen hier insbesondere solche mit 4 bis 20 C-Atomen in Betracht, z.B. iso-Buten, 1-Octen oder 1-Dodecen.
Beispiele für Sulfamidocarbonsäuren (l) sind Sulfonamide der Anthranilsäure sowie Neutralisationsprodukte von Sulfamidocarbonsäuren mit Alkanolaminen, Dialkanolaminen oder Trialkanolaminen.
Als Ammoniumsalze von Sulfonsäuren (m) kommen beispielsweise entsprechende Salze der 2-Aminoethansulfonsäure (Taurin) in Betracht.
Selbstverständlich können die genannten Zusatzstoffe nicht nur alleine sondern auch in Mischung zugegeben werden, wobei natürlich die Summe der Anteile der Komponenten des NBM 100 Gew.-% ergeben muß.
Weiter kann der Einsatz der NBM sowohl in gelöster als auch in fester Form erfolgen, wobei diese im Falle

der dithionithaltigen NBM bevorzugt ist.

Üblicherweise gibt man je Liter des Färbeflottenvolumens 0,1 g 25 bis 5,0 g des NBM, bezogen auf die Trokkenmasse, zu.

Im Hochtemperaturausziehverfahren erfolgt die Färbung der Textilien beispielsweise bei 120 - 140°C in Druckkesseln, welche erst bei Temperaturen unter 100°C druckfrei und damit gefahrlos zu öffnen sind. Andererseits läuft die reduktive Nachreinigung der polyesterhaltigen Textilien in der Färbeflotte erst oberhalb von etwa 50°C mit zufriedenstellender Geschwindigkeit ab. Der bevorzugte Temperaturbereich während der Nachbehandlung liegt daher bei 50 - 100°C.

Die Zeitdauer der Nachbehandlung betrug in den aufgeführten Ausführungsbeispielen 5 - 20 min, doch können sich unter betrieblichen Bedingungen davon abweichende Dauern ergeben. Diese werden von Parametern wie etwa der Temperatur, dem Volumen der Färbe-flotte und damit zusammenhängend der Effizienz der Zumischung des NBM aber natürlich auch durch die Natur der verwendeten Farbmittel beeinflußt.

Im Falle der dithionithaltigen NBM erfolgt deren verfahrensgemäßer Einsatz bevorzugt in fester Form. Hier ist jedoch auch die Verwendung von wäßrigen Lösungen möglich.

Bei Einsatz von Dispergiermitteln und/oder Tensiden in flüssigem oder gelöstem Zustand muß dies einer festen Formulierung des NBM nicht entgegenstehen. Sofern die restlichen Komponenten in fester Form vorliegen, was bei Dithioniten, säurebindenden Substanzen, Hydroxyketonen und Korrosionsinhibitoren in der Regel der Fall sein wird, reicht deren Bindekapazität für die Aufnahme eines flüssigen oder gelösten Bestandteils, vor allem im hier beanspruchten Anteil von 2 bis 20 Gew.-% an der Gesamtmischung, meist für die Bereitstellung eines granulierbaren und/oder gut rieselfähigen Produkts aus.

Beispiele:

Flottenfärbungen/Färbungen

Die Färbungen wurden durchgeführt, indem man texturiertes Polyestergewebe mit 4 Gew.-% des Farbstoffs zusammen mit 1 g eines handelsüblichen Naphthalinsulfonsäure-Formaldehyd-Kondensates (Tamol®NOP) als Dispergiermittel und 0,5 g Nitrilotriessigsäuresalz (Trilon®A92) als Komplexbildner - beide Zusätze jeweils bezogen auf einen Liter des wäßrigen Färbebades - in essigsaurer Flotte mit einem pH-Wert von 4,5 - 5,0 mit einer Aufheizrate von 1°C/min von anfänglich 70°C auf 130°C erhitzte und bei dieser Endtemperatur 60 min beließ. Das Verhältnis von polyesterhaltigem Gewebe (trocken) in Kilogramm zu Färbebadvolumen in Liter, das sogenannte Flottenverhältnis, betrug 1 : 20.

Nachbehandlung

Nach beendetem Färbeschritt wurde das NBM bei der Temperatur T der auskühlenden Färbeflotte zugegeben und für die Zeit t einwirken gelassen. Die unterschiedlichen Temperaturen T in °C und Einwirkdauern t in Minuten sind in Tabelle 1 aufgeführt. Für die unterschiedlichen NBM sind in Tabelle 1 Konzentrationen angegeben, die als Menge des NBM in Gramm auf das Volumen der Färbe-flotte (entspricht dem Volumen aus Färbebad einschließlich Polyestergewebe) in Liter zu verstehen sind. Schließlich wurde das Färbebad abgelassen und das gefärbte Gewebe 5 min mit kaltem Wasser nachgespühlt.

Waschtest

Das nach dem Färbeschritt entweder nur gespülte oder zusätzlich noch nachbehandelte, gefärbte Polyestergewebe wurde zusammen mit einem Stück weißem Polyamidgewebe desselben Trockengewichts im Wasserbad bei einem pH-Wert von 5 und einer Temperatur von 70°C für 30 min erhitzt. Das Flottenverhältnis von Gewebe zu Bad betrug wiederum 1 : 20. Die Wirksamkeit der Nachbehandlung wurde anhand des Grades der Anfärbung des ursprünglich weißen Polyamidgewebes visuell beurteilt. War dieses weiß geblieben, so fand kein Ausbluten des gefärbten Polyestergewebes statt (Beurteilung ++). Entsprechend wurde geringe bzw. starke Anfärbung des Polyamidgewebes beobachtet, wenn das Polyestergewebe geringfügig bzw. stark ausblutete (Beurteilungen + bzw. -).

Tabelle 1

| | | | | | Nachbehandlungsmittel (NBM) | | | | | | | | | |
|---|---|---|---|---|---|---|---|---|---|---|---|---|---|---|
| Bsp. | Farbstoff | T (°C) | t (min) | Konz. NBM (g/l) | Reduktionsmittel | Gew.% | Säurebindende Substanz | Gew.% | Hydroxyketon | Gew.% | Dispergiermittel bzw. Tensid | Gew.% | End-pH-Wert | Ergebnis** |
| 1 | ROT | 80 | 10 | 1.0 | Hydrosulfit | 50 | $Na_2CO_3$ | 30 | Saccharose | 20 | — | — | 8.0 | + |
| 1a* | ROT | 80 | 10 | 1.0 | Hydrosulfit | 100 | — | — | — | — | — | — | 3.0 | - |
| 2 | BLAU | 70 | 20 | 2.0 | Hydrosulfit | 50 | $Na_2CO_3$ | 10 | Glucose | 40 | — | — | 5.5 | + |
| 2a* | BLAU | — | — | — | — | — | — | — | — | — | — | — | 3.0 | - |
| 2b* | BLAU | 70 | 20 | 2.0 | Hydrosulfit | 100 | — | — | — | — | — | — | — | - |
| 3 | BLAU | 80 | 20 | 3.0 | Hydrosulfit | 35 | $Na_2CO_3$ | 20 | Glucose | 25 | Tamol NOP / Oxoalkohol | 10/ 10 | 7.0 | ++ |
| 4 | ROT | 90 | 10 | 1.5 | Hydrosulfit | 40 | $K_2CO_3$ | 20 | Saccharose | 30 | Tamol NOP | 10 | 7.0 | ++ |
| 5a* | ROT | 80 | 15 | 1.0 | $HOCH_2SO_2Na$ | 100 | — | — | — | — | — | | 5.0 | + |
| 5b* | ROT | 80 | 15 | 1.0 | $HOCH_2SO_2Na$/ $HOCH_2SO_3Na$ | 50/50 | — | — | — | — | — | | 5.0 | + |
| 6a* | BLAU | 80 | 15 | 1.0 | $HOCH_2SO_2Na$ | 100 | — | — | — | — | — | | 5.0 | + |

Tabelle 1 (Fortsetzung)

| | | | | | Nachbehandlungsmittel (NBM) | | | | | | | | | |
|---|---|---|---|---|---|---|---|---|---|---|---|---|---|---|
| Bsp. | Farbstoff | T (°C) | t (min) | Konz. NBM (g/l) | Reduktionsmittel | Gew.% | Säure-bindende Substanz | Gew.% | Hydroxy-keton | Gew.% | Dispergier-mittel bzw. Tensid | Gew.% | End-pH-Wert | Ergebnis** |
| 6b* | BLAU | 80 | 15 | 1.0 | $HOCH_2SO_2Na$/ $HOCH_2SO_3Na$ | 50/50 | — | — | — | — | — | | 5.0 | + |
| 7 | BLAU | 80 | 15 | 2.0 | $N(CH_2SO_2Na)_3$/ $N(CH_2SO_3Na)_3$ | 50/50 | — | — | — | — | — | — | 7.0 | + |
| 8 | ROT | 70 | 15 | 2.0 | $HN(CH_2SO_2Na)_2$/ $Na_2SO_3$ | 80/20 | — | — | — | — | — | — | 6.0 | ++ |
| 9a | BLAU | 90 | 10 | 1.0 | $N(CH_2SO_2Na)_3$ | 50 | — | — | Glucose | 30 | Tamol NOP | 20 | 5.0 | ++ |
| 9b | BLAU | 90 | 10 | 2.0 | $N(CH_2SO_2Na)_3$ | 50 | — | — | Glucose | 30 | Tamol NOP | 20 | 5.0 | ++ |
| 10a | ROT | 70 | 15 | 1.5 | Na-2-Ethylhexyl-aminomethan-sulfinat | 100 | — | — | — | — | — | — | 6.0 | ++ |
| 10b | ROT | 70 | 20 | 2.0 | Na-2-Ethylhexyl-aminomethan-sulfinat | 100 | — | — | — | — | — | — | 6.0 | ++ |
| 11 | BLAU | 70 | 20 | 2.0 | Na-2-Ethylhexyl-aminomethansulfi-nat | 100 | — | — | — | — | — | — | 6.0 | ++ |

Tabelle 1 (Fortsetzung)

| | | | | | Nachbehandlungsmittel (NBM) | | | | | | | | | |
|---|---|---|---|---|---|---|---|---|---|---|---|---|---|---|
| Bsp. | Farbstoff | T (°C) | t (min) | Konz. NBM (g/l) | Reduktionsmittel | Gew.% | Säurebindende Substanz | Gew.% | Hydroxyketon | Gew.% | Dispergiermittel bzw. Tensid | Gew.% | End-pH-Wert | Ergebnis** |
| 12 | ROT | 80 | 15 | 0.66[1] | $N(CH_2SO_2Na)_3$/ $HOCH(CH_3)SO_3Na$ | 90/10 | — | — | — | — | — | — | 6.0 | ++ |
| 13 | BLAU | 80 | 15 | 0.66[1] | $N(CH_2SO_2Na)_3$/ $HOCH(CH_3)SO_3Na$ | 90/10 | — | — | — | — | — | — | 6.0 | ++ |
| 14 | ROT | 80 | 15 | 0.88[2] | $N(CH_2SO_2Na)_3$/ $N(CH_2SO_3Na)_3$/ $HOCH(CH_3)SO_3Na$ | 40/50/10 | — | — | — | — | — | — | 6.0 | ++ |
| 15 | BLAU | 80 | 15 | 0.88[2] | $N(CH_2SO_2Na)_3$/ $N(CH_2SO_3Na)_3$/ $HOCH(CH_3)SO_3Na$ | 40/50/10 | — | — | — | — | — | — | 6.0 | ++ |
| 16 | ROT | 80 | 15 | 0.88[2] | $N(CH_2SO_2Na)(CH_2SO_3Na)_2$ | 100 | — | — | — | — | — | — | 6.0 | ++ |
| 17 | ROT | 80 | 15 | 0.44[2] | $N(CH_2SO_2Na)(CH_2SO_3Na)_2$ | 100 | — | — | — | — | — | — | 6.0 | ++ |

ROT: C.I. Disperse Red 54:1
BLAU: C.I. Disperse Blue 56
*: Vergleichsbeispiel; bei 2a erfolgte keine Nachbehandlung
1: 2 g/l einer 33 %igen wäßrigen Lösung
2: 2 g/l einer 44 %igen wäßrigen Lösung

**: ++ *kein Ausbluten*
+ geringes Ausbluten
– starkes Ausbluten

Die in Tabelle 1 aufgeführten Beispiele zeigen, daß durch das erfindungsgemäße Verfahren hervorragende Echtheitsverbesserungen von polyesterhaltigen Texilien erzielt werden. Zudem läßt sich die bisher übliche Zeit- und energieintensive Schrittfolge der Färbung, Wäsche nach der Färbung, alkalischen reduktiven Nachreinigung sowie nochmaligen abschließenden Nachwäsche verkürzen auf den reinen Färbeschritt gefolgt von der Zugabe des NBM in die Färbeflotte und einen abschließenden Waschschritt. Ein weiterer Vorteil des erfindungsgemäßen Verfahrens besteht in der deutlichen Reduzierung der Salzfrachten (überwiegend $Na_2SO_4$) gegenüber der herkömmlichen Verfahrensweise, auch bei Verwendung dithionithaltiger NBM (Beispiele 1 - 4).

## Patentansprüche

1. Verfahren zur reduktiven Nachreinigung von gefärbten, polyesterhaltigen Textilien, **dadurch gekennzeichnet, daß** man der sauren Färbeflotte als Nachbehandlungsmittel eine Mischung zusetzt, welche als Komponenten enthält

   a) mindestens eine Verbindung der Formel (I)

   $$A_m[(CR^1R^2)_mSO_2M]_{p,q} \qquad (I)$$

   worin bedeuten

   | | |
   |---|---|
   | A | $NR^3_{3-q}$ oder $OR^4_{2-p}$ |
   | $R^1$,$R^2$,$R^4$ | Wasserstoff, $C_1$-$C_6$-Alkyl |
   | $R^3$ | identische oder verschiedene Reste ausgewählt aus der Gruppe Wasserstoff, $C_1$-$C_{20}$-Alkyl, $C_3$-$C_8$-Cycloalkyl ggf. substituiert durch ein bis drei $C_1$-$C_4$-Alkylreste |
   | M | Äquivalent eines Ammonium-, Alkali- oder Erdalkaliions |
   | m | 0 oder 1 |
   | p | 1 oder 2 |
   | q | 1, 2 oder 3 |

   wobei mindestens einer der Reste $R^1$, $R^2$, $R^4$ ein $C_1$-$C_6$-Alkyl ist, wenn A für $OR^4_{2-p}$ steht, und wobei für den Fall, daß m gleich 0 ist:

   p, q den Wert 2 annimmt

   und die Verbindung der Formel (I) in Mischung mit einer Menge an säurebindender Substanz verwendet wird, welche ausreicht, um den pH-Wert der Färbeflotte um 1 bis 3 Einheiten zu erhöhen,

   b) gegebenenfalls mindestens eine Verbindung der Formel (II)

   $$A[(CR^1R^2)SO_3M]_{p,q} \qquad (II)$$

   worin A, $R^1$, $R^2$, $R^3$, $R^4$, M, p und q dieselbe allgemeine Bedeutung wie in Formel (I) besitzen, wobei die Auswahl dieser Variablen im konkreten Einzelfall für die Verbindungen der Formeln (I) und (II) nicht gleich sein muß

   c) und gegebenenfalls weitere Zusatzstoffe.

2. Verfahren nach Anspruch 1 **dadurch gekennzeichnet, daß** man für m gleich 0 die Verbindung der Formel (I) in

Mischung mit säurebindenden Substanzen aus der Gruppe Ammonium-, Alkalioder Erdalkalihydrogencarbonat, -carbonat, -oxid, -hydroxid, -hydrogensulfit, -sulfit, -hydrogenphosphat oder -phosphat zusetzt.

3. Verfahren nach Anspruch 1, **dadurch gekennzeichnet, daß** man für m gleich 0 die Verbindung der Formel (I) und die säurebindende Substanz in einem Gewichtsverhältnis von 3:1 bis 1:1 einsetzt.

4. Verfahren nach Anspruch 1, **dadurch gekennzeichnet, daß** man mindestens eine Verbindung der Formel (I) mit mindestens einer Verbindung der Formel (II) in einem molaren Mischungsverhältnis von 20:1 bis 1:20 einsetzt.

5. Verfahren nach den Ansprüchen 1 bis 4, **dadurch gekennzeichnet, daß** das Nachbehandlungsmittel als weitere Zusatzstoffe enthält mindestens

ca) ein Hydroxyketon oder ein sich aus mehreren gleichen oder verschiedenen Hydroxyketonen ableitendes oligo- oder polymeres Produkt und/oder

cb) ein Dispergiermittel oder Tensid oder eine Mischung daraus und/oder

cc) ein Ammonium-, Alkali- oder Erdalkalisulfit, -hydrogensulfit oder -disulfit.

6. Verfahren nach Anspruch 5, **dadurch gekennzeichnet, daß** die Zusatzstoffe in einem Anteil von

ca) 5-60 Gew.-%,

cb) 2-50 Gew.-%,

cc) 5-30 Gew.-%

an der Gesamtmischung des Nachbehandlungsmittels enthalten sind.

7. Verfahren nach den Ansprüchen 1, 3, 4 bis 6, **dadurch gekennzeichnet, daß** man als Nachbehandlungsmittel eine Mischung zusetzt, welche als Komponenten enthält

a) mindestens ein Dithionit $S_2O_4M_2$ in Mischung mit einer säurebindenden Substanz, ausgewählt aus der Gruppe der Alkali- und Erdalkalicarbonate oder -hydrogencarbonate,

b) gegebenenfalls mindestens eine Verbindung (II) ausgewählt aus der Gruppe $HOCH(CH_3)SO_3Na$, $H_2MCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ und Natriumsalze der 1-, 2-, 3-, 4-, 5-Ethylhexylaminomethansulfonsäure und -ethansulfonsäure und

c) gegebenenfalls mindestens einen weiteren Zusatzstoff aus der Gruppe der Mono- oder Disaccharide und/oder ein Tensid und/oder ein Dispergiermittel.

8. Verfahren nach den Ansprüchen 1, 4 bis 6, **dadurch gekennzeichnet, daß** man als Nachbehandlungsmittel eine Mischung zusetzt, welche als Komponenten enthält

a) mindestens eine Verbindung (I) ausgewählt aus der Gruppe $HOCH(CH_3)SO_2Na$, $H_2NCH_2SO_2Na$, $HN(CH_2SO_2Na)_2$, $N(CH_2SO_2Na)_3$, $H_2NCH(CH_3)SO_2Na$, $HN(CH(CH_3)SO_2Na)_2$, $N (CH(CH_3)SO_2Na)_3$ und Natriumsalze der 1-, 2-, 3-, 4-, 5-Ethylhexylaminomethansulfinsäure und -ethansulfinsäure und

b) gegebenenfalls mindestens eine Verbindung (II) ausgewählt aus der Gruppe $HOCH(CH_3)SO_3Na$, $H_2NCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ und Natriumsalze der 1-, 2-, 3-, 4-, 5-Ethylhexylaminomethansulfonsaure und -ethansulfonsäure und

c) gegebenenfalls mindestens einen weiteren Zusatzstoff aus der Gruppe der Mono- oder Disaccharide und/oder ein Tensid und/oder ein Dispergiermittel.

9. Verfahren nach den Ansprüchen 1 bis 7, **dadurch gekennzeichnet, daß** man das Nachbehandlungsmittel in fester Form einsetzt.

**10.** Verfahren nach den Ansprüchen 1 bis 9, **dadurch gekennzeichnet, daß** man das Nachbehandlungsmittel in einer auf die Trokkenmasse bezogenen Menge von 0.1 bis 5.0 g je Liter des Färbeflotten- oder Waschbadvolumens einsetzt.

**Claims**

**1.** A process for reduction clearing dyed polyester textiles, which comprises adding, to the acidic dyeing liquor, an aftertreatment composition comprising as components

a) at least one compound of the formula (I)

$$A_m[(CR^1R^2)_mSO_2M]_{p,q} \qquad (I)$$

where

| | |
|---|---|
| A | is $NR^3_{3-q}$ or $OR^4_{2-p}$ |
| $R^1$,$R^2$,$R^4$ | are each hydrogen or $C_1$-$C_6$-alkyl |
| $R^3$ | represents identical or different radicals selected from the group consisting of hydrogen, $C_1$-$C_{20}$-alkyl, $C_3$-$C_8$-cycloalkyl opt. substituted by from one to three $C_1$-$C_4$-alkyl radicals |
| M | is the equivalent of an ammonium, alkali metal or alkaline earth metal ion |
| m | is 0 or 1 |
| p | is 1 or 2 |
| q | is 1, 2 or 3 |

subject to the provisos that at least one of $R^1$, $R^2$, $R^4$ is $C_1$-$C_6$-alkyl when A is $OR^4_{2-p}$ and that, if m is 0:

p, q are each 2
and
the compound of the formula (I) is used together with sufficient acid acceptor to raise the pH of the dyeing liquor by from 1 to 3 units,

b) optionally at least one compound of the formula (II)

$$A[(CR^1R^2)SO_3M]_{p,q} \qquad (II)$$

where A, $R^1$, $R^2$, $R^3$, $R^4$, M, p and q are each as defined for the formula (I), although the specific choice of these variables does not have to be the same for the compounds of the formulae (I) and (II)

c) and optionally further additives.

**2.** A process as claimed in claim 1, wherein m is 0 and the compound of the formula (I) is added together with acid acceptors selected from the group consisting of the bicarbonates , carbonates, oxides, hydroxides, bisulfites, sulfites, hydrogenphosphates or phosphates of ammonium, alkali metals or alkaline earth metals.

**3.** A process as claimed in claim 1, wherein m is 0 and the compound of the formula (I) and the acid acceptor are used in a weight ratio within the range from 3:1 to 1:1.

**4.** A process as claimed in claim 1, wherein at least one compound of the formula (I) is used with at least one compound

of the formula (II) in a molar mixing ratio within the range from 20:1 to 1:20.

**5.** A process as claimed in any of claims 1 to 4, wherein the aftertreatment composition includes as further additives at least

ca) a hydroxyketone or an oligomer or polymer derived from a plurality of identical or different hydroxyketones, and/or

cb) a dispersant or surfactant or a mixture thereof and/or

cc) an ammonium, alkali metal or alkaline earth metal sulfite, bisulfite or disulfite.

**6.** A process as claimed in claim 5, wherein the additives are present in the total mixture of the aftertreatment composition in a proportion of

ca) 5-60 % by weight,

cb) 2-50 % by weight,

cc) 5-30 % by weight.

**7.** A process as claimed in any of claims 1, 3 and 4 to 6, wherein the aftertreatment composition comprises as components

a) at least one dithionite $S_2O_4M_2$ in a mixture with an acid acceptor selected from the group of the alkali metal and alkaline earth metal carbonates or bicarbonates,

b) optionally at least one compound (II) selected from the group consisting of $HOCH(CH_3)SO_3Na$, $H_2NCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ and sodium salts of 1-, 2-, 3-, 4- and 5-ethylhexylamino-methanesulfonic acid and -ethanesulfonic acid, and

c) optionally at least one further additive from the group of the mono- or disaccharides and/or a surfactant and/or a dispersant.

**8.** A process as claimed in any of claims 1 and 4 to 6, wherein the aftertreatment composition comprises as components

a) at least one compound (I) selected from the group consisting of $HOCH(CH_3)SO_2Na$, $H_2NCH_2SO_2Na$, $HN(CH_2SO_2Na)_2$, $N(CH_2SO_2Na)_3$, $H_2NCH(CH_3)SO_2Na$, $HN(CH(CH_3)SO_2Na)_2$, $N(CH(CH_3)SO_2Na)_3$ and sodium salts of 1-, 2-, 3-, 4- and 5-ethylhexylamino-methanesulfinic acid and-ethanesulfinic acid, and

b) optionally at least one compound (II) selected from the group consisting of $HOCH(CH_3)SO_3Na$, $H_2NCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ and sodium salts of 1-, 2-, 3-, 4- and 5-ethylhexylamino-methanesulfonic acid and -ethanesulfonic acid, and

c) optionally at least one further additive from the group of the mono- or disaccharides and/or a surfactant and/or a dispersant.

**9.** A process as claimed in any of claims 1 to 7, wherein the aftertreatment composition is used in solid form.

**10.** A process as claimed in any of claims 1 to 9, wherein the aftertreatment composition is used in an amount based on the solids content within the range from 0.1 to 5.0 g per liter of the dyeing liquor or wash bath volume.

## Revendications

1. Procédé pour le rinçage complémentaire réducteur de textiles contenant des polyesters et teints, **caractérisé par le fait que** l'on ajoute au bain de teinture acide, en tant que produit de traitement complémentaire, un mélange contenant en tant que composants

   a) au moins un composé de formule I

   $$A_m[(CR^1R^2)_mSO_2m]_{p,q} \qquad (I)$$

   dans laquelle

   A représente $NR^3_{3-q}$ ou $OR^4_{2-p}$

   $R^1$, $R^2$, $R^4$ représentent l'hydrogène ou des groupes alkyle en C1-C6,

   les symboles $R^3$, ayant des significations identiques ou différentes, représentent chacun l'hydrogène, un groupe alkyle en C1-C20, cycloalkyle en C3-C8 éventuellement substitué par un à trois groupes alkyle en C1-C4,

   M représente un équivalent d'un ion ammonium, alcalin ou alcalino-terreux,

   m est égal à 0 ou 1,

   p est égal à 1 ou 2,

   q est égal à 1, 2 ou 3,

   l'un au moins des symboles $R^1$, $R^2$, $R^4$ représentant un groupe alkyle en C1-C6 lorsque A représente $OR^4_{2-p}$, et, lorsque m est égal à 0,

   p et q sont égaux à 2

   et
   le composé de formule I est utilisé en mélange avec une substance captant les acides en quantité suffisante pour accroître le pH du bain de teinture de 1 à 3 unités,

   b) le cas échéant, au moins un composé de formule II

   $$A[(CR^1R^2)SO_3M]_{p,q} \qquad (II)$$

   dans laquelle A, $R^1$, $R^2$, $R^3$, $R^4$, M, p et q ont les significations générales indiquées en référence à la formule I, le choix de ces variables, dans chaque cas particulier, n'étant pas obligatoirement identique pour les composés de formules I et II,

   c) et le cas échéant d'autres additifs.

2. Procédé selon la revendication 1, **caractérisé par le fait que**, lorsque m est égal à 0, on ajoute le composé de formule I en mélange avec des substances captant les acides prises dans le groupe des bicarbonates, des carbonates, des oxydes, des hydroxydes, des hydrogénosulfites, des sulfites, des hydrogénophosphate et des phosphates d'ammonium, alcalins ou alcalino-terreux.

3. Procédé selon la revendication 1, **caractérisé par le fait que**, lorsque m est égal à 0, on utilise le composé de formule I et la substance captant les acides à des proportions relatives en poids de 3 : 1 à 1 : 1.

4. Procédé selon la revendication 1, **caractérisé par le fait que** l'on utilise au moins un composé de formule I avec

au moins un composé de formule II à des proportions molaires de mélange de 20 : 1 à 1 : 20.

**5.** Procédé selon les revendications 1 à 4, **caractérisé par le fait que** le produit de traitement complémentaire contient, en tant qu'autres additifs, au moins

ca) une hydroxycétone ou un produit oligomère ou polymère dérivant de plusieurs hydroxycétones identiques ou différentes et/ou

cb) un agent dispersant ou tensio-actif ou un mélange de tels agents et/ou

cc) un sulfite, un hydrogénosulfite ou un disulfite d'ammonium, alcalin ou alcalino-terreux.

**6.** Procédé selon la revendication 5, **caractérisé par le fait que** les additifs sont contenus en proportions de

ca) 5 à 60 % en poids
cb) 2 à 50 % en poids
cc) 5 à 30 % en poids,

par rapport au mélange total du produit de traitement complémentaire.

**7.** Procédé selon les revendications 1, 3, 4 à 6, **caractérisé par le fait que** l'on ajoute, en tant que produit de traitement complémentaire, un mélange contenant en tant que composants

a) au moins un dithionite $S_2O_4M_2$ en mélange avec une substance captant les acides choisie dans le groupe des carbonates et bicarbonates alcalins et alcalino-terreux,

b) le cas échéant au moins un composé II choisi parmi $HOCH(CH_3)SO_3Na$, $H_2NCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ et les sels de sodium des acides 1-, 2-, 3-, 4-, 5-éthylhexylamino-méthanesulfoniques et -éthanesulfoniques et

c) le cas échéant au moins un autre additif du groupe des mono- ou di-saccharides et/ou un agent tensio-actif et/ou un agent dispersant.

**8.** Procédé selon les revendications 1, 4 à 6, **caractérisé par le fait que** l'on ajoute, en tant que produit de traitement complémentaire, un mélange contenant en tant que composants

a) au moins un composé I chois parmi $HOCH(CH_3)SO_2Na$, $H_2NCH_2SO_2Na$, $HN(CH_2SO_2Na)_2$, $N(CH_2SO_2Na)_3$, $H_2NCH(CH_3)SO_2Na$, $HN(CH(CH_3)SO_2Na)_2$, $N(CH(CH_3)SO_2Na)_3$ et les sels de sodium des acides 1-, 2-, 3-, 4-, 5-éthylhexylamino-méthanesulfiniques et -éthanesulfiniques et

b) le cas échéant au moins un composé II choisi parmi $HOCH(CH_3)SO_3Na$, $H_2NCH_2SO_3Na$, $HN(CH_2SO_3Na)_2$, $N(CH_2SO_3Na)_3$, $H_2NCH(CH_3)SO_3Na$, $HN(CH(CH_3)SO_3Na)_2$, $N(CH(CH_3)SO_3Na)_3$ et les sels de sodium des acides 1-, 2-, 3-, 4-, 5-éthylhexylamino-méthanesulfoniques et -éthanesulfoniques et

c) le cas échéant au moins un autre additif du groupe des mono- et di-saccharides et/ou un agent tensio-actif et/ou un agent dispersant.

**9.** Procédé selon les revendications 1 à 7, **caractérisé par le fait que** le produit de traitement complémentaire est mis en oeuvre à l'état solide.

**10.** Procédé selon les revendications 1 à 9, **caractérisé par le fait que** l'on utilise le produit de traitement complémentaire en quantité, exprimée en masse sèche, de 0,1 à 5,0 g par litre du bain de teinture ou du bain de lavage.